# EUROPEAN PATENT APPLICATION

(11) **EP 4 782 102 A1**
(43) Date of publication of application: **29.07.2026**
(21) Application number: 24867084.6
(22) Date of filing: 24.07.2024
(51) Int. Cl.: B01L 3/00, G01N 33/53

(54) **MICRO-FLUIDIC CHIP AND TESTING METHOD THEREOF**

(30) Priority: 22.09.2023 CN 202311230921; 22.09.2023 CN 202311230967
(71) Applicant: Beijing Micvic Biotech, Co., Ltd., Beijing 101200 (CN); Shandong Mic-Vic Biotech Co., Ltd., Weihai, Shandong 264210 (CN)
(72) Inventor: LI, Songjing, Harbin, Heilongjiang 150001 (CN); YANG, Xiaohui, Tianjin 300203 (CN); LIU, Huiyu, Weihai, Shandong 264210 (CN); LI, Huiqiang, Tianjin 300203 (CN)
(74) Representative: Ran, Handong
(86) International application number: PCT/CN2024/107224
(87) International publication number: WO 2025/060667

(57) **Abstract**

The present disclosure relates to a micro-fluidic chip, comprising a substrate; a cover piece, the cover piece comprising a groove extending in the length direction; and a micro-channel defined by the substrate and the groove, wherein the groove of the cover piece comprises a channel area, the channel area is sequentially provided with a sample adding area, a reaction area and a control area in the length direction, and the sample adding area, the reaction area and the control area are in flid communication with each other, wherein a control valve is arranged in the control area, the control valve is configured to control the flow of liquid in the reaction area to the control area, the height of the reaction area is lower than that of the control area, and the width of the reaction area is smaller than the width of the control area. The control valve can enable the liquid in the reaction area to flow towards the control area at the required time, so that the time in the sample reaction area is controlled, and the liquid flows into the control area after full reaction and is not retained in a channel of the reaction area.

## Description

### Cross-Reference

The present application claims priorities to Chinese Patent Application No. 202311230967.4, filed with the China National Intellectual Property Administration on September 22, 2023, and entitled "A Microfluidic Chip," and Chinese Patent Application No. 202311230921.2, entitled "A Microfluidic Chip and a Detection Method Thereof," the entire contents of which are incorporated herein by reference.

### Technical Field

The present invention relates to the field of in-vitro diagnostic technology, and specifically relates to a microfluidic chip and a detection method thereof.

### Background Art

A microfluidic chip is the main platform for implementing microfluidic technology, which can integrate basic operational units such as sample preparation, reaction, separation, and detection in biological, chemical, and medical analysis processes onto a very small chip. The entire analysis process is automatically completed through micro-channels to achieve various functions of conventional chemical or biological laboratories. Microfluidic chips have the advantages of being lightweight, using a small amount of sample and reagents, having a fast reaction speed, being capable of large-scale parallel processing, and being disposable, and they have huge potential in the fields of biology, chemistry, and medicine. In recent years, they have developed into a brand-new interdisciplinary research field involving biology, chemistry, medicine, fluids, electronics, materials, machinery, and other disciplines.

A fixed volume of reaction liquid is generally added to a microfluidic chip, and by utilizing its special structure, the sample after loading is allowed to flow in the micro-channel under capillary action and finally reach a waste liquid area, thereby completing an automatic reaction process, which is then coordinated with an optical analyzer for detection to analyze detection results.

In known microfluidic chips, a loading hole and a buffer hole are usually both arranged upstream of a labeling zone, while the labeling zone is arranged upstream of a detection zone. Therefore, after a sample passes through the loading hole, it first reacts in the labeling zone and then enters the detection zone. For samples with high viscosity, the signal molecules in the labeling zone cannot be pushed to the detection zone by the sample itself, and a subsequent buffer is required for pushing. However, this method dilutes the sample, resulting in inaccurate detection results, and other substances contained in the sample may also interfere with the signal molecules in the labeling zone.

Regarding the flow and control of the sample in the microfluidic chip, it is an important focus of microfluidic chips. A known microfluidic chip with controllable reaction time involves embedding a water-absorbent material at an outlet of the sample. The water-absorbent material contacts the micro-channel to adsorb the sample stagnant in the channel. However, this method cannot control the timing at which the sample in the channel is adsorbed, which easily leads to incomplete reaction in the channel.

Furthermore, another known microfluidic chip comprises a micro-channel with a defined height and width formed by enclosing a substrate and a cover plate. A sample fluid can flow toward an outlet of the micro-channel under the capillary action of the micro-channel. The height of the top wall of the micro-channel is lower than the height of the contact surface between the substrate and the cover plate. However, this method requires ensuring the injection volume of the sample liquid, and liquid leakage may still occur during the operation process, making the detection results inaccurate.

### Summary of the Invention

The object of the present invention, aimed at the deficiencies of the prior art, is to provide a microfluidic chip and a detection method thereof to solve the technical problems raised in the above background art.

On one hand, to achieve the above object, the present invention provides a microfluidic chip, comprising a substrate, a cover plate, and a micro-channel enclosed and formed by the substrate and the cover plate. The micro-channel comprises a buffer loading zone, a reaction zone, and a control zone, wherein the buffer loading zone, the reaction zone, and the control zone are sequentially in communication along a length direction. A movable water-absorbent material is arranged in the control zone, and the water-absorbent material is capable of contacting or being away from the reaction zone. Furthermore, the reaction zone comprises a labeling zone, a detection zone, and a sample loading zone, and the detection zone and the sample loading zone are both arranged on the same side of the labeling zone.

In some examples, the cover plate is provided with a groove along the length direction, and the groove and the substrate enclose to form the micro-channel.

In some examples, a detection site and a quality control site are sequentially arranged in the detection zone along the length direction, and the number of the detection site and the quality control site is one or more.

In some examples, the labeling zone comprises an antibody/antigen labeled with a signal molecule; the detection site of the detection zone is coated with an antibody/antigen capable of binding with a target analyte. The target analyte is capable of simultaneously binding with the antibody/antigen of the labeling zone and the detection site to form a double antigen/antibody sandwich complex.

The quality control site of the detection zone is coated with an antibody/antigen capable of binding with the antibody/antigen labeled with the signal molecule of the labeling zone.

In some examples, a braking hole is arranged at a side of the control zone opposite to the reaction zone. The braking hole is a rectangular through-hole penetrating through the substrate and the cover plate, and at least partially extends to the water-absorbent material in the control zone. An end of the water-absorbent material away from the reaction zone is provided with a through-hole, and the through-hole is within the braking hole.

In some examples, the buffer loading zone is provided with a buffer hole communicating upper and lower surfaces of the cover plate, and the sample loading zone is provided with a loading hole communicating the upper and lower surfaces of the cover plate.

In some examples, the sample loading zone and the labeling zone are respectively arranged on two sides of the detection zone.

In some examples, the sample loading zone is arranged between the labeling zone and the detection zone.

In some examples, the antigen/antibody labeled with the signal molecule is an antigen/antibody molecule labeled with a signal molecule.

On the other hand, the present invention further provides a detection method for a microfluidic chip, wherein the microfluidic chip as described above is applied for detection. The method comprises at least the following steps:
Adding a sample into the micro-channel through the sample loading zone, and keeping the water-absorbent material away from the reaction zone;
After the sample flows back through the detection zone under capillary action and satisfies a first determination condition, moving the water-absorbent material to contact the reaction zone to suck excessive sample until flow interruption occurs, and allowing the sample retained in the micro-channel to react with the detection zone; then, the water-absorbent material separates from or continues to contact the reaction zone;
After reacting for a second time period, adding a buffer into the micro-channel through the buffer loading zone. The buffer passes through the labeling zone under capillary action to wash the antigen/antibody coated with signal molecules in the labeling zone and then enters the detection zone. When the signal molecules in the reaction zone channel converge with the sample, moving the water-absorbent material in the control zone to contact the reaction zone to adsorb the excessive sample into the control zone through the water-absorbent material;
After the excessive sample in the channel is sucked away by the water-absorbent material and the signal molecules of the labeling zone are dissolved to fill the reaction zone channel, moving the water-absorbent material away from the reaction zone to disconnect the contact between the water-absorbent material and the reaction zone channel;
After reacting for a third time period, moving the water-absorbent material in the control zone to contact the detection zone to adsorb the excessive liquid comprising the signal molecules into the control zone through the water-absorbent material;
Measuring a signal value of the detection zone.

In some examples, the first determination condition comprises: a first time period has elapsed, or a sample liquid volume is greater than or equal to a predetermined amount, or a signal intensity of the signal molecules in the detection zone is greater than or equal to a threshold.

In some examples, the first time period is within a range of 0 to 60 seconds, the predetermined amount is within a range of 15 µL to 50 µL, and the threshold is higher than a reasonable background range of the chip. For different signal molecules and corresponding detection equipment, the range of signal intensity varies. The reasonable background range referred to herein is an intensity greater than the background signal, i.e., significantly showing the intensity of the corresponding signal molecule.

In some examples, the second time period is within a range of 10 seconds to 300 seconds, and the third time period is within a range of 30 seconds to 5 minutes.

The beneficial effects of the microfluidic chip and the detection method thereof obtained through the above technical solutions are:
1. Since the sample loading zone is closer to the detection zone, the flow distance of the sample in the micro-channel is reduced, thereby decreasing the sample dosage. The sample can react with the detection zone immediately after being added, and the buffer is used to push signal molecules after the sample has reacted in the detection zone. This avoids dilution of the sample caused by directly pushing the sample with the buffer, resulting in high sensitivity and more accurate detection results.
2. Detection can be directly performed on whole blood or samples with high viscosity, offering a wide range of sample applicability. Signal molecules in the labeling zone are pushed into the reaction zone by the buffer rather than being directly pushed by the sample, which avoids contamination of the channel surface by whole blood or high-viscosity samples that might adhere to the channel and affect subsequent detection accuracy.
3. Excessive sample is thoroughly adsorbed by the water-absorbent material after reacting in the reaction zone, preventing other substances in the sample from affecting the signal molecules.
4. The hook effect of immune reaction can be overcome, avoiding situations where the target analyte content in certain samples is too high (far exceeding the number of antigens/antibodies labeled with signal molecules). This prevents the target analyte from occupying all reaction sites in the reaction zone, which would otherwise result in little or no immune complex formed by target analytes and signal molecules reacting with antigens/antibodies in the detection zone, thus ensuring accurate signal measurement.

In yet another aspect, the present invention provides a microfluidic chip, comprising: a substrate; a cover plate comprising a groove extending along a length direction; and a micro-channel enclosed and formed by the substrate and the groove. The groove of the cover plate comprises a channel area, which is sequentially provided with a loading area, a reaction zone, and a control zone along the length direction. The loading area, the reaction zone, and the control zone are in fluid communication with each other. A control valve is arranged in the control zone and is configured to control the flow of liquid from the reaction zone toward the control zone. The height of the reaction zone is lower than the height of the control zone, and the width of the reaction zone is smaller than the width of the control zone.

In some examples, the control zone gradually narrows from its middle toward the reaction zone.

In some examples, the control valve comprises a movable fluid suction element and a braking hole. The fluid suction element is capable of contacting or being away from the reaction zone. The braking hole is arranged at a side away from the reaction zone and is a rectangular through-hole penetrating through the substrate and the cover plate. An end of the fluid suction element away from the reaction zone is provided with a through-hole, which is within the braking hole.

In some examples, the control zone is provided with a locking part configured to maintain the fluid suction element at a position away from the reaction zone.

In some examples, the locking part comprises a convex part arranged along a width direction, and the fluid suction element is correspondingly provided with a concave part matching the convex part.

In some examples, the cover plate is provided with a retention part at a junction between the control zone and the reaction zone. The retention part at least partially extends into the reaction zone such that when the fluid suction element contacts the reaction zone channel, at least a portion of the fluid suction element is inserted into the retention part.

In some examples, the retention part is a tooth part constructed as a downwardly recessed arc extending into the reaction zone, and a lower end of the arc is provided with a serrated or wavy structure. A side of the fluid suction element close to the reaction zone gradually narrows and is provided with a notch in the middle, such that when the fluid suction element contacts the reaction zone channel, the notch is inserted into the tooth part.

In some examples, the retention part is an inclined slot slanting toward the reaction zone.

In some examples, the fluid suction element comprises a water-absorbent material, wherein the water-absorbent material comprises at least one of a desiccant, an antioxidant, or a moisture indicator.

In some examples, the loading area is provided with a loading hole communicating upper and lower surfaces of the cover plate, and the height of the loading hole at the lower surface of the cover plate is at the same level as the height of the reaction zone.

In some examples, the groove is rectangular, the shape of the substrate matches the groove, and the substrate is placed within the groove and is at the same level as a surface of the cover plate.

In some examples, a channel area, a barrier area, and a pressing area are respectively arranged from the middle toward the outer sides along a width direction within the groove of the cover plate. The heights of the channel area and the barrier area are higher than the height of the pressing area, such that the substrate is placed within the groove on the lower surface of the cover plate to contact the pressing area and forms a gap with the channel area and the barrier area.

In some examples, the barrier area is arranged at a periphery of the loading area and/or the reaction zone of the channel area, and in the length direction, the barrier area covers at least a portion of the reaction zone, or covers the loading area and/or the reaction zone.

In some examples, the substrate is coated with a hydrophobic material at a position corresponding to the barrier area in the groove of the cover plate.

In some examples, the reaction zone and the barrier area are at the same level, and the barrier area is also coated with the hydrophobic material.

In some examples, the substrate is subjected to laser treatment at a position corresponding to the barrier area in the groove of the cover plate to form a hydrophobic surface.

In some examples, the reaction zone and the barrier area are at the same level, and the barrier area is subjected to laser treatment to form a hydrophobic surface.

In some examples, the height of the reaction zone is lower than the height of the barrier area, such that a sample does not enter the barrier area due to gravity when flowing along the reaction zone.

The beneficial effects of the microfluidic chip obtained through the above technical solutions are:
1. A control valve is arranged in the control zone to control the flow of liquid from the reaction zone toward the control zone at a required timing. This allows for control of the sample's residence time in the reaction zone, ensuring sufficient reaction before flowing into the control zone without remaining in the reaction zone channel.
2. The tooth part of the control valve ensures that when the fluid suction element is inserted to contact the reaction zone, its top is inserted into the arc-shaped structure of the reaction zone, and its bottom contacts the serrated or wavy structure. Fibers inside the fluid suction element cross-link upon contact with the serrated or wavy structure, causing the bottom of the fluid suction element to be compressed and thickened for better contact with the reaction zone channel, achieving the goal of adsorbing stagnant samples.
3. The control valve can be used multiple times in a single experiment to open or close the passage between the reaction zone and the control zone as needed. Liquid in the channel can be adsorbed multiple times at appropriate intervals, increasing detection accuracy.
4. The substrate is coated with hydrophobic material or laser-treated at positions corresponding to the barrier area, restricting the bottom of the sample to flow within the corresponding reaction zone during loading without entering the barrier area.
5. The barrier area on the cover plate is higher than the reaction zone, ensuring the sample flows within the reaction zone due to gravity.
6. When the barrier area and reaction zone have the same height, applying hydrophobic material or laser treatment to the barrier area also restricts the sample to the reaction zone.

### Description of Drawings

In order to explain the technical solutions of the embodiments of the present disclosure more clearly, the drawings of the embodiments will be briefly introduced below. Obviously, the drawings in the description below only relate to some embodiments of the present disclosure, rather than limiting the present disclosure.
FIG. 1 is a schematic top view structure diagram of a microfluidic chip according to at least one embodiment of the present invention;
FIG. 2 is a schematic structure diagram of a cover plate of a microfluidic chip according to at least one embodiment of the present invention;
FIG. 3 is a schematic cross-sectional structure diagram taken along line A-A in FIG. 2;
FIG. 4 is an enlarged view of part B in FIG. 2, which illustrates a retention part of a tooth part;
FIG. 5 is an enlarged view of part C in FIG. 3, which illustrates a cross-sectional structure of the retention part;
FIG. 6 is a schematic structure diagram of a cover plate of a microfluidic chip according to another embodiment of the present invention;
FIG. 7 is a schematic cross-sectional structure diagram taken along line D-D in FIG. 6;
FIG. 8 is an enlarged view of part E in FIG. 6;
FIG. 9 is an enlarged view of part F in FIG. 7;
FIG. 10 is a schematic structure diagram of a substrate according to at least one embodiment of the present invention;
FIG. 11 is a schematic side cross-sectional structure diagram of a microfluidic chip according to at least one embodiment of the present invention, illustrating a groove of a cover plate;
FIG. 12 is a schematic side cross-sectional structure diagram of a microfluidic chip according to yet another embodiment of the present invention, illustrating a groove of a cover plate;
FIG. 13 is a schematic structure diagram of a cover plate at a position of a groove according to another embodiment of the present invention;
FIG. 14 is a schematic side cross-sectional structure diagram of a microfluidic chip according to yet another embodiment of the present invention, illustrating a groove of a cover plate;
FIG. 15 is a schematic structure diagram of a microfluidic chip according to at least one embodiment of the present invention;
FIG. 16 is a schematic cross-sectional structure diagram of a microfluidic chip according to at least one embodiment of the present invention;
FIG. 17 is a schematic structure diagram of a microfluidic chip according to another embodiment of the present invention.

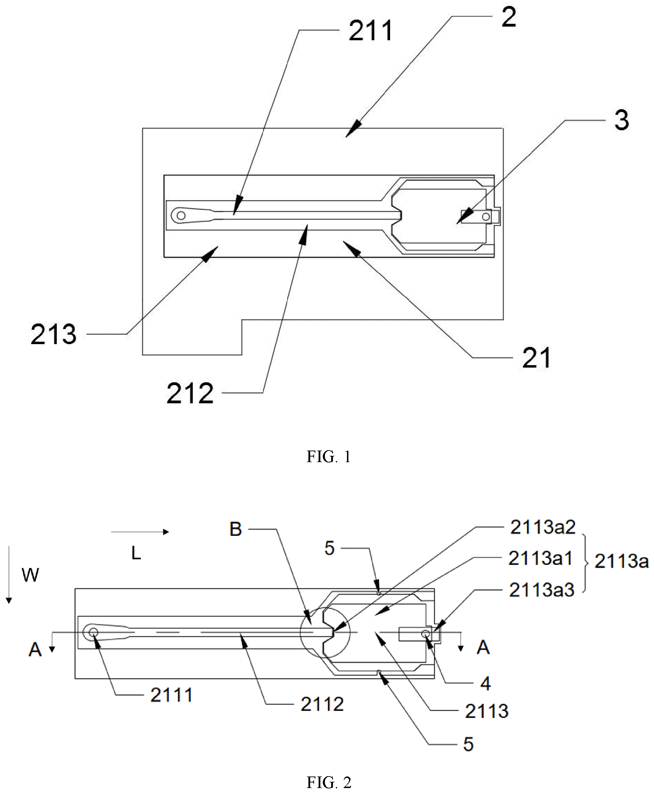

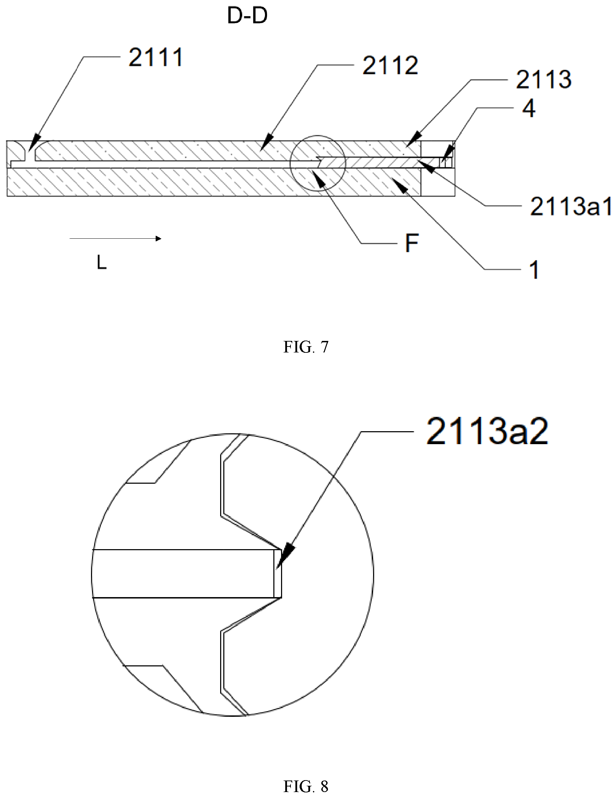

Reference numerals in FIG. 1 to FIG. 14: 1-substrate; 2-cover plate; 21-groove; 211-channel area; 2111-loading area; 2112-reaction zone; 2113-control zone; 2112a-control valve; 2113a1-fluid suction element; 2113a2-tooth part; 2113a3-braking hole; 212-barrier area; 213-pressing area; 3-micro-channel; 4-through-hole; 5-locking part.

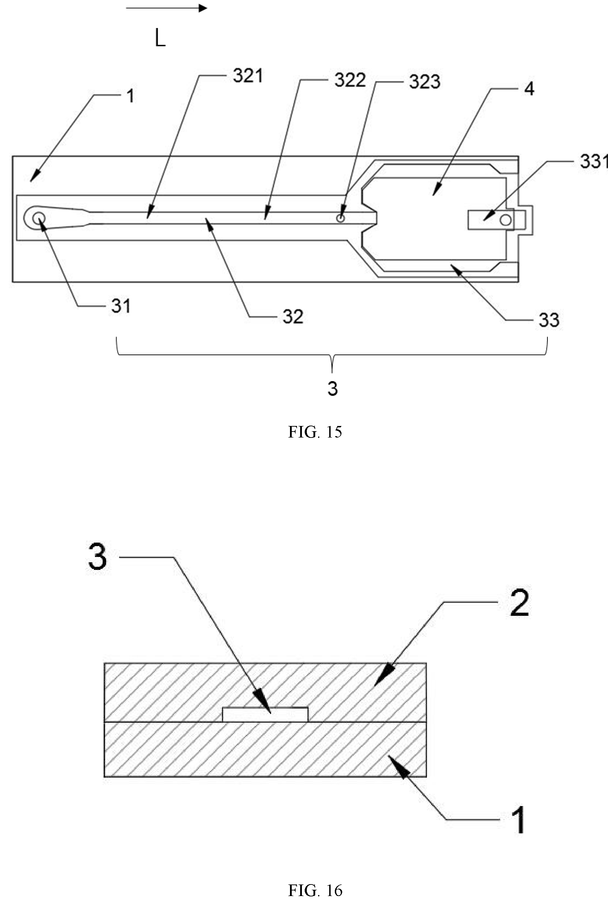

Reference numerals in FIG. 15 to FIG. 17: 1-substrate; 2-cover plate; 3-micro-channel; 4-water-absorbent material; 31-buffer loading zone; 32-reaction zone; 33-control zone; 321-labeling zone; 322-detection zone; 323-sample loading zone.

### Detailed Description

In view of the defects in the prior art, the present invention provides a novel microfluidic chip and a detection method thereof. The present invention will be described in detail below in conjunction with the accompanying drawings. It should be noted that in the drawings, the same reference numerals are given to components having substantially the same or similar structures and functions, and repetitive descriptions thereof will be omitted.

Compared with the embodiments shown in the drawings, feasible embodiments within the scope of protection of the present invention may have fewer components, other components not shown in the drawings, different components, differently arranged components, or differently connected components, etc. In addition, without departing from the concept of the present invention, two or more components in the drawings may be implemented in a single component, or a single component shown in the drawings may be implemented as a plurality of separate components. As shown in FIG. 1 to FIG. 5, a microfluidic chip according to at least one embodiment of the present invention comprises a substrate 1, a cover plate 2, and a micro-channel formed by enclosing the substrate 1 and the cover plate 2. A groove 21 is provided in a middle of a lower surface of the cover plate 2, and the groove 21 extends along a length direction L.

It should be noted that the length direction L in the drawings refers to a direction along the length of the microfluidic chip, which is exemplarily a horizontal rightward direction in the figures. The width direction W in the drawings refers to a direction along the width of the microfluidic chip, which is exemplarily a vertical downward direction in the figures.

As shown in FIG. 1, a channel area 211, a barrier area 212, and a pressing area 213 are respectively arranged from the middle toward the outer sides along the width direction W within the groove 21 of the cover plate 2. Heights of the channel area 211 and the barrier area 212 are higher than a height of the pressing area 213, such that the substrate 1 is placed within the groove 21 on the lower surface of the cover plate 2 to contact the pressing area 213 and forms a gap with the channel area 211 and the barrier area 212.

As shown in FIG. 2, the channel area 211 is sequentially provided with a loading area 2111, a reaction zone 2112, and a control zone 2113 along a liquid flow direction (length direction L). The loading area 2111, the reaction zone 2112, and the control zone 2113 are in communication with each other. The height of the reaction zone 2112 is lower than a height of the control zone 2113, and a width of the control zone 2113 is greater than a width of the reaction zone 2112. In conjunction with features of a control valve 2113a and a retention part 2113a2 described later, the height difference between the reaction zone 2112 and the control zone 2113 facilitates stable maintenance of a fluid suction element 2113a1 at a contact position with the reaction zone 2112. Furthermore, the width of the reaction zone 2112 being smaller than the width of the control zone 2113 helps excessive liquid to be absorbed into the control zone 2113 and accommodated therein. Exemplarily, the control zone 2113 may gradually narrow from its middle toward the reaction zone 2112.

In the present embodiment, a control valve 2113a is arranged in the control zone 2113, and the control valve 2113a is configured to control a flow of liquid from the reaction zone 2112 toward the control zone 2113. This allows the liquid in the reaction zone 2112 to flow toward the control zone at a required timing, thereby controlling a residence time of a sample in the reaction zone 2112 to ensure sufficient reaction before flowing to the control zone 2113 without remaining in the reaction zone 2112 channel.

Specifically, the control valve 2113a may be implemented in various ways. For example, in an embodiment shown in FIG. 2 to FIG. 5, the control valve 2113a may comprise a braking hole 2113a3 and a movable fluid suction element 2113a1. The fluid suction element 2113a1 is capable of contacting or being away from the reaction zone 2112. When the fluid suction element 2113a1 contacts the reaction zone 2112, the fluid suction element 2113a1 can suck liquid from the reaction zone 2112 into the control zone 2113. Conversely, when the fluid suction element 2113a1 is away from or not in contact with the reaction zone 2112, the liquid will remain in the reaction zone 2112 without flowing into the control zone 2113.

Referring again to FIG. 2, in order to prevent accidental contact between the fluid suction element 2113a1 and the reaction zone 2112, the control zone 2113 is provided with a locking part 5. The locking part 5 is configured to maintain the fluid suction element 2113a1 at a position away from the reaction zone 2112. In the present embodiment, the locking part 5 comprises convex parts arranged along the width direction W, and the fluid suction element 2113a1 is correspondingly provided with concave parts matching the convex parts. The convex parts may be arc-shaped or rectangular, and may be at the same position or staggered in the length direction.

Alternatively, the locking part 5 may also be other possible detent structures, such as a snap structure or an elastic reset element that biases the fluid suction element 2113a1 toward the reaction zone 2112.

The cover plate 2 is provided with a retention part 2113a2 at a junction between the control zone 2113 and the reaction zone 2112. The retention part 2113a2 at least partially extends into the reaction zone 2112 such that when the fluid suction element 2113a1 contacts the channel of the reaction zone 2112, at least a portion of the fluid suction element 2113a1 is inserted into the retention part 2113a2.

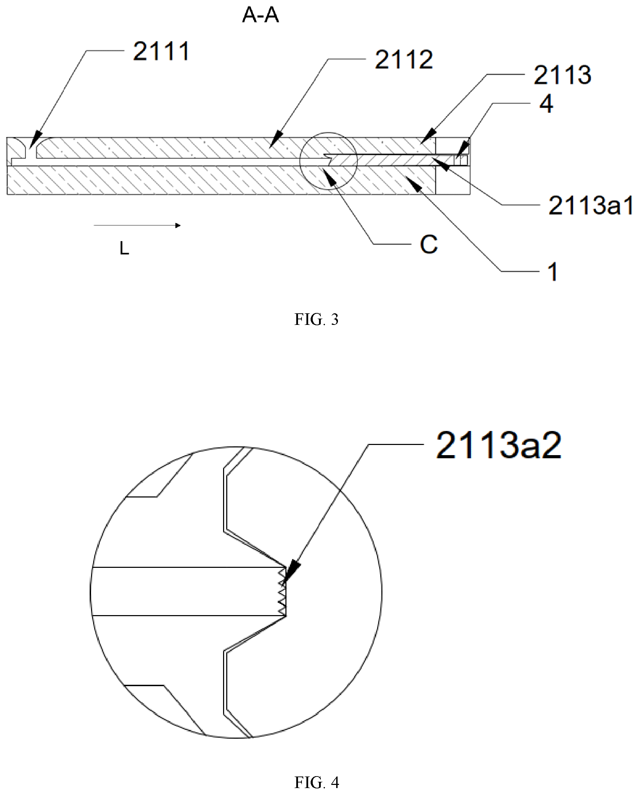

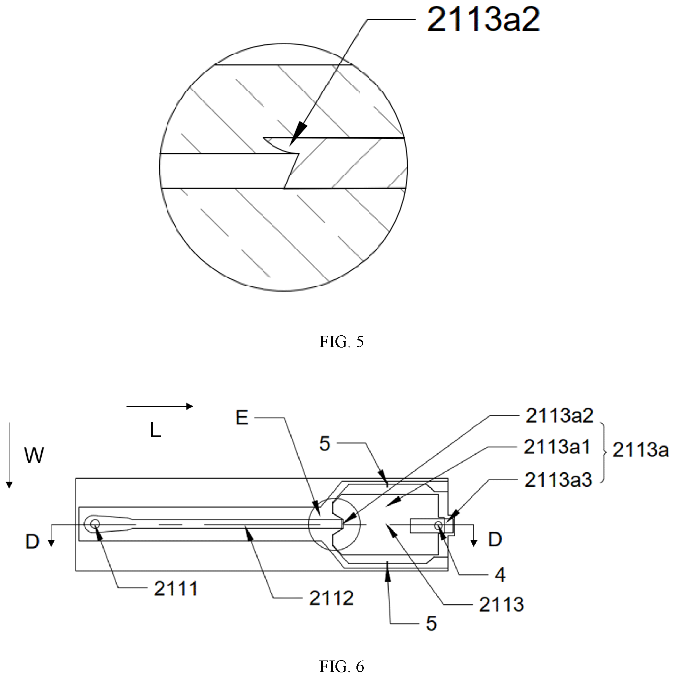

As shown in FIG. 4 and FIG. 5, the retention part 2113a2 may be a tooth part constructed as a downwardly recessed arc extending into the reaction zone 2112, and a lower end of the arc may be provided with serrations. A side of the fluid suction element 2113a1 close to the reaction zone 2112 gradually narrows, and a notch is provided in a middle of the side close to the reaction zone 2112 such that when the fluid suction element 2113a1 contacts the channel of the reaction zone 2112, the notch is inserted into the tooth part.

Alternatively, in an embodiment not shown, the lower end of the arc may also be set as a wavy structure. Distinct from serrations, the wavy structure may be an undulating curve.

Therefore, when the fluid suction element 2113a1 is inserted into the control zone 2113 to contact the reaction zone 2112, its top is inserted into the arc-shaped structure of the reaction zone 2112, and its bottom contacts the serrations at the bottom of the arc-shaped structure. Fibers inside the fluid suction element 2113a1 cross-link upon contact with the serrations, causing the bottom of the fluid suction element 2113a1 to be compressed and thickened for better contact with the channel of the reaction zone 2112, achieving the goal of adsorbing stagnant samples in the channel.

The movement of the fluid suction element 2113a1 can be achieved through structures of the braking hole 2113a3 and the through-hole 4. As shown in FIG. 2, the braking hole 2113a3 is a rectangular through-hole penetrating through the substrate and the cover plate. An end of the fluid suction element 2113a1 away from the reaction zone may be provided with a through-hole 4, and the through-hole 4 is arranged within a range of the braking hole 2113a3. For example, a braking rod can be inserted into the braking hole 2113a3 and pass through the through-hole 4 on the fluid suction element 2113a1 to drive the fluid suction element 2113a1 to move, so that the other end of the fluid suction element 2113a1 contacts or leaves the reaction zone 2112. Adsorption of the liquid in the reaction zone 2112 onto the fluid suction element 2113a1 can be controlled at a suitable timing.

Exemplarily, FIG. 6 to FIG. 9 also show a cover plate structure of a microfluidic chip according to another embodiment of the present invention. It should be noted that the following description of the embodiment mainly focuses on its differences from the aforementioned embodiments, and descriptions of the same or similar features will be omitted. Different from the previous embodiments, the present embodiment adopts a simpler retention part and another structure of the locking part. As shown in FIG. 6 to FIG. 9, the retention part 2113a2 is an inclined slot slanting toward the reaction zone 2112. The inclined slot can also accommodate and cross-link the fiber structure of the fluid suction element 2113a1, and although its effect might not be as good as the tooth part, its processing is simpler and the cost is lower.

In addition, in this embodiment, the convex parts of the locking part 5 are rectangular, and the concave parts of the fluid suction element 2113a1 are concave parts matching the rectangular convex parts. Since the rectangular width of the concave part is small, it appears like a straight line in FIG. 6. This is because it is suggested that the width of the rectangular concave part should not be too large, otherwise it will affect the movement of the fluid suction element 2113a1. Alternatively, the control valve 2113a may also adopt structures such as pneumatic valves or plunger pumps known in the art to control the flow of liquid, which will not be repeated here. Exemplarily, the fluid suction element 2113a1 is a water-absorbent material, and the water-absorbent material may comprise or have added at least one of a desiccant, an antioxidant, or a moisture indicator.

As another example, the water-absorbent material may adopt polyester fiber, water-absorbent resin, water-absorbent gelatin, papermaking wood pulp, or other materials with water-absorbent characteristics.

Exemplarily, the reaction zone 2112 is sequentially provided with a labeling zone, a detection zone, and a quality control zone along the length direction L, which can also be referred to as a sample flow direction.

The groove 21 in the middle of the cover plate 2 is rectangular. The shape of the substrate 1 matches the groove 21, and the substrate 1 is placed within the groove 21 and is at the same level as the surface of the cover plate 2.

The loading area 2111 is provided with a loading hole communicating the upper and lower surfaces of the cover plate 2. A height of the loading hole at the lower surface of the cover plate 2 is at the same level as the height of the reaction zone 2112, such that a sample flows through the loading hole to the reaction zone 2112 on the right side.

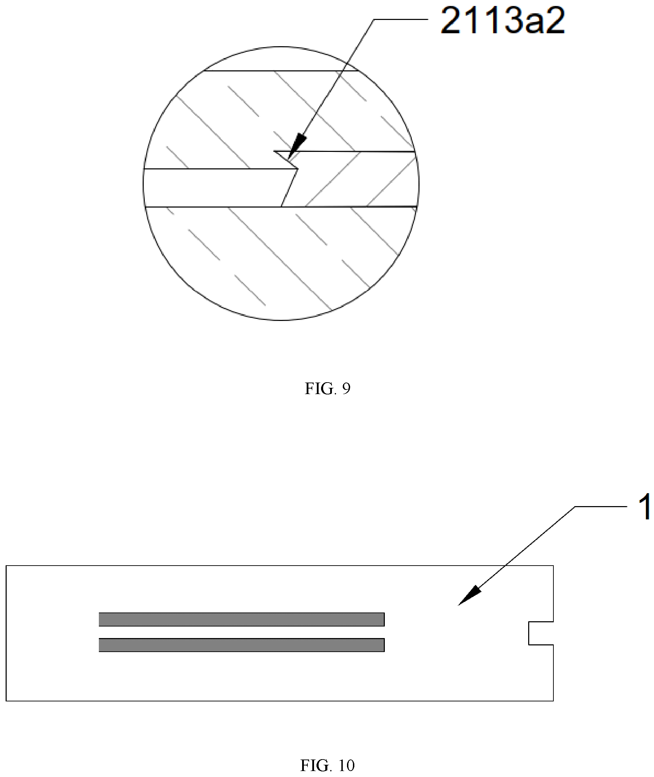

The barrier area 212 is arranged at the periphery of the reaction zone 2112 in the channel area 211, and the barrier area 212 covers the entire reaction zone 2112 in the length direction L. As shown in FIG. 10, the substrate 1 may be coated with a hydrophobic material at a position corresponding to the barrier area 212 in the groove 21 of the cover plate 2, such that during a loading process, the bottom of the sample is restricted to flow within the corresponding reaction zone 2112 without entering the barrier area 212.

Alternatively, in an embodiment not shown, the barrier area 212 may also cover only a portion of the reaction zone 2112, or be arranged only at the loading area 2111 and cover the loading area 2111, or be arranged at the periphery of the loading area 2111 and the reaction zone 2112 and cover the loading area 2111 and the reaction zone 2112.

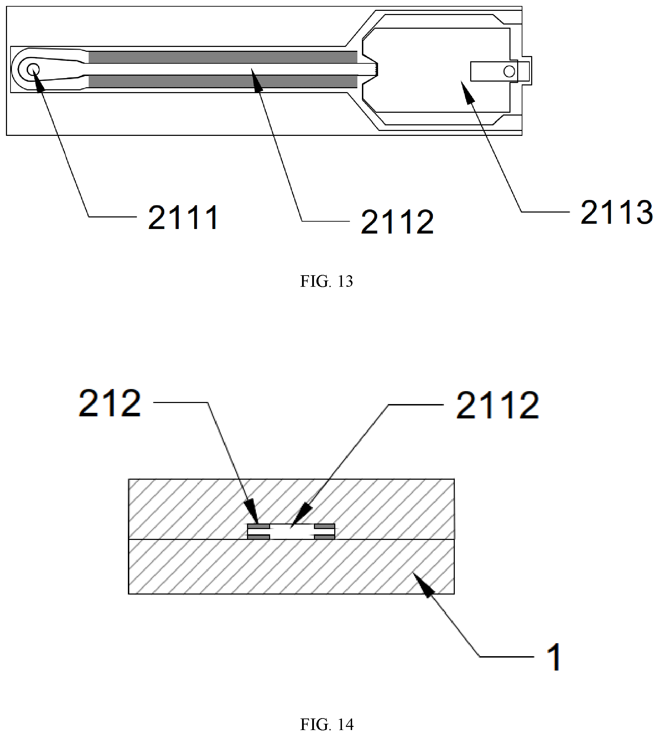

As shown in FIG. 13 and FIG. 14, the reaction zone 2112 and the barrier area 212 are at the same level, and the barrier area 212 is also coated with a hydrophobic material to restrict the sample to flow within the reaction zone 2112. By coating the barrier area 212 with the hydrophobic material, it is ensured that the sample liquid is restricted within the channel of the reaction zone 2112 without leaking or flowing to a non-reaction zone (such as the barrier area 212) during reaction, thereby improving reaction sensitivity.

Exemplarily, the hydrophobic material may adopt polytetrafluoroethylene ink, silane coupling agent ink, fluorinated polymer ink, or other materials with hydrophobic characteristics.

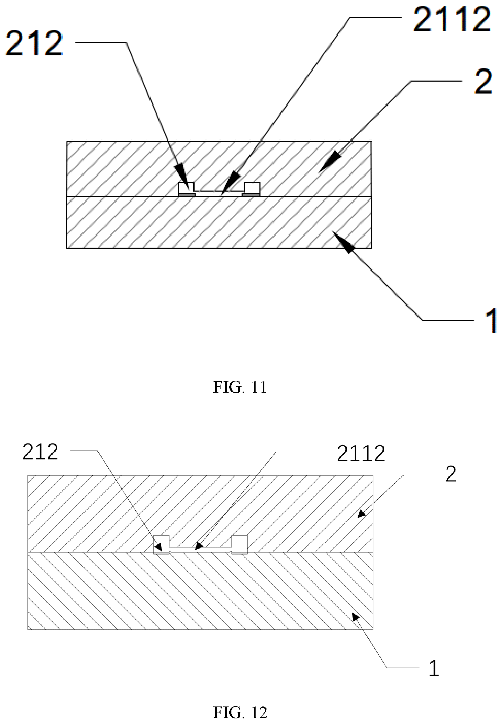

As shown in FIG. 11, the height of the reaction zone 2112 is lower than the height of the barrier area 212, so that the sample does not enter the barrier area 212 due to gravity when flowing along the reaction zone 2112. Therefore, it is not necessary to add a hydrophobic material in the barrier area 212 to also restrict the sample to flow within the reaction zone 2112.

It should be noted that the solid-colored filled portions in FIG. 11 and FIG. 13 show the coated hydrophobic material, which is shown with a larger thickness only for the purpose of convenience of description. In fact, the coated hydrophobic material can be a very thin layer of coating.

Alternatively, instead of coating a hydrophobic material at the positions in the above FIG. 10 and FIG. 11, the original hydrophilic layer can be removed by laser treatment to form a hydrophobic surface, as shown in FIG. 12. The laser treatment can be, for example, laser sintering or laser marking. The position and size of the laser treatment can be consistent with those of the hydrophobic material coated in the previous embodiments.

After the laser treatment, the original hydrophilic layer is removed, and a recessed structure is formed at the same time. The recessed structure itself has hydrophobic properties. In addition, due to the high temperature of the laser, a side of the recessed structure close to the channel will be melted and form a protruding part, further forming a physical barrier that obstructs the liquid from flowing into the recessed structure.

Alternatively, in an embodiment where the reaction zone 2112 and the barrier area 212 are at the same level, the barrier area 212 can also be directly subjected to laser treatment to form a hydrophobic surface. At this time, the substrate 1 can either be coated with a hydrophobic material at a position corresponding to the barrier area 212, or be similarly subjected to laser treatment as shown in FIG. 12.

The following shows several experimental examples of applying the microfluidic chip of the present invention to verify and illustrate technical effects claimed by the present invention.

### Example 1

Experimental purpose: To compare the influence on interpretation results after adding a control valve in a waste liquid area (control zone).
1. Material preparation
A microfluidic chip No. 1 before improvement and a microfluidic chip No. 2 with a control valve after improvement. Compared with the chip No. 1 before improvement, the chip No. 2 after improvement has a control valve added to the waste liquid area (control zone) at the end of the reaction zone. Both of the above are produced by Shandong Micro-Diagnostic Biotechnology Co., Ltd.
RSV clinical samples obtained from relevant hospitals.
A fluorescence immunoassay analyzer produced by Shandong Micro-Diagnostic Biotechnology Co., Ltd., a timer (such as a stopwatch), and a pipette.

2. Coating sites
Coating site one is located in the detection zone and is coated with an RSV antibody.
Coating site two is located in the quality control zone and is coated with a secondary antibody.
A fluorescence labeling zone is fixed with dried fluorescence-labeled conjugated antibodies.

3. Detection method
3.1 Microfluidic chip with a control valve after improvement (chip No. 2) The improved microfluidic chip with a control valve (chip No. 2) was placed flat on a laboratory bench. RSV standard solution samples with concentrations of 100 ng/ml, 5 ng/ml, and 1 ng/ml were respectively added into the loading hole of the microfluidic chip. After timing for 90 seconds, the control valve was used to bring the water-absorbent material into contact with the reaction zone, so that the liquid in the reaction zone flowed toward the control zone. The chip was interpreted using a fluorescence immunoassay analyzer, and detection results of IgE samples were recorded. Each sample was repeatedly detected twice, and a detection signal value of each sample, a signal value of the quality control zone (C line), and a signal value of the detection zone (T line) were recorded.
3.2 Microfluidic chip before improvement (chip No. 1) The microfluidic chip before improvement (chip No. 1) was placed flat on a laboratory bench. RSV standard solution samples with concentrations of 100 ng/ml, 5 ng/ml, and 1 ng/ml were respectively added into the loading hole of the microfluidic chip. After the samples reacted, they naturally flowed to the waste liquid area at the end of the reaction zone and were adsorbed by the water-absorbent material. The chip was interpreted using a fluorescence immunoassay analyzer, and detection results of IgE samples were recorded. Each sample was repeatedly detected twice, and a detection signal value of each sample, a signal value of the quality control zone (C line), and a signal value of the detection zone (T line) were recorded.

4. Results
As shown in Table 1, by using the control valve to control reaction for 90 seconds and then connecting the reaction zone with the control zone, the chip No. 2 after improvement allows for sufficient reaction of the sample in the reaction zone, and detected signal values of the T line and the C line are both higher than those of the chip No. 1 before improvement.

**Table 1 Sample detection results before and after improvement of the microfluidic chip Table**

| Chip No. 1 | | | | |
|---|---|---|---|---|
| RSV Sample Concentration | T-line Signal Value | C-line Signal Value | T/C | Mean Value |
| 100 ng/ml | 287789 | 415226 | 0.693 | 0.699 |
| | 380953 | 540457 | 0.705 | |
| 5 ng/ml | 31670 | 568231 | 0.056 | 0.055 |
| | 24008 | 437303 | 0.055 | |
| 1 ng/ml | 0 | 583349 | 0.000 | 0.002 |
| | 2075 | 569394 | 0.004 | |

| Chip No. 2 | | | | |
|---|---|---|---|---|
| RSV Sample Concentration | T-line Signal Value | C-line Signal Value | T/C | Mean Value |
| 100 ng/ml | 544218 | 623340 | 0.873 | 0.884 |
| | 412621 | 461087 | 0.895 | |
| 5 ng/ml | 54600 | 597830 | 0.091 | 0.086 |
| | 57531 | 706961 | 0.081 | |
| 1 ng/ml | 23048 | 606442 | 0.038 | 0.035 |
| | 19907 | 619136 | 0.032 | |

Experimental conclusion: By adding a control valve in the waste liquid area (control zone) at the end of the reaction zone to control the flow of liquid from the reaction zone toward the control zone, and thereby controlling the residence time of the sample in the reaction zone, the liquid in the reaction zone flows toward the control zone after sufficient reaction, resulting in higher detected signal values and higher accuracy.

### Example 2

Experimental purpose: To compare a water absorption speed after adding a retention part at the end of the reaction zone and its influence on interpretation results.
1. Material preparation
An IgE microfluidic chip No. 3 before improvement and an IgE microfluidic chip No. 4 with a tooth part after improvement. Compared with the chip No. 3 before improvement, the chip No. 4 after improvement has a retention part (a tooth part in the example) added at a cover plate of a channel at the end of the reaction zone (near the water-absorbent material end). Both are produced by Shandong Micro-Diagnostic Biotechnology Co., Ltd.
An IgE clinical serum sample S1 obtained from a relevant hospital.
A fluorescence immunoassay analyzer produced by Shandong Micro-Diagnostic Biotechnology Co., Ltd., a timer (such as a stopwatch), and a pipette.

2. Coating sites
Coating site one is located in the detection zone and is coated with an IgE antibody.
Coating site two is located in the quality control zone and is coated with a secondary antibody.
A fluorescence labeling zone is fixed with dried IgE fluorescence-labeled conjugated antibodies.

3. Detection method
3.1 IgE microfluidic chip with a tooth part after improvement The improved microfluidic chip with a tooth part (chip No. 4) was placed flat on a laboratory bench. 35 µL of sample was added into the loading hole of the microfluidic chip. After timing for 90 seconds, the water-absorbent material was brought into contact with the channel to suck water. A time taken for the water-absorbent material to suck the liquid until flow interruption was recorded. The chip was interpreted using a fluorescence immunoassay analyzer, and detection results of the IgE sample were recorded. Each sample was repeatedly detected 3 times, and a detection signal value of each sample, a peak value of the quality control zone (C line), and a peak value of the detection zone (T line) were recorded.
3.2 IgE microfluidic chip before improvement The microfluidic chip before improvement (chip No. 3) was placed flat on a laboratory bench. 35 µL of sample was added into the loading hole of the microfluidic chip. After timing for 90 seconds, the water-absorbent material was brought into contact with the channel to suck water. A time taken for the water-absorbent material to suck the liquid until flow interruption was recorded. The chip was interpreted using a fluorescence immunoassay analyzer, and detection results of the IgE sample were recorded. Each sample was repeatedly detected 3 times, and a detection signal value of each sample, a peak value of the quality control zone (C line), and a peak value of the detection zone (T line) were recorded.

4. Results
As shown in Table 2, water absorption times of the chip No. 4 are all within 2 minutes and 30 seconds, while a water absorption time of the chip No. 3 is about 3 minutes. There is no significant difference in terms of signal values. The improved water absorption time can save 30 seconds compared with the original chip.

**Table 2 Sample detection results before and after improvement of the microfluidic chip**

| Chip No. 3 | | | | | |
|---|---|---|---|---|---|
| Sample | Item | Water Absorption Time | Detection Signal Value | C-line Peak Value | T-line Peak Value |
| S1 | IgE | 2min 55sec | 983.73 | 65 | 330 |
| | | 3min 02sec | 1065.85 | 77 | 465 |
| | | 2min 48sec | 972.50 | 62 | 315 |

| Chip No. 4 | | | | | |
|---|---|---|---|---|---|
| Sample | Item | water Absorption Time | Detection Signal Value | C-line Peak Value | T-line Peak Value |
| S1 | IgE | 1min 50sec | 998.52 | 80 | 380 |
| | | 2min 06sec | 980.65 | 64 | 377 |
| | | 2min 18sec | 1004.19 | 80 | 436 |

Experimental conclusion: Adding a tooth part at the end of the reaction zone will have a flow-guiding effect on the liquid and increase a contact surface between the liquid and the water-absorbent material. The water absorption speed will be faster than that of the original structure, and there is almost no influence on interpretation results. An interpretation speed of an instrument can be optimized.

### Example 3

Experimental purpose: To contrastively verify the influence on interpretation results after coating a hydrophobic material on two sides of positions of an upper cover channel corresponding to a substrate.
1. Material preparation
   A four-test (RSV/FluA/FluB/COVID-19) microfluidic chip No. 5 before improvement and a four-test (same test items as above) microfluidic chip No. 6 coated with a hydrophobic material. Compared with the chip No. 5, the substrate of the chip No. 6 is sprayed with a hydrophobic coating at positions corresponding to the channel after being assembled with the upper cover, preventing sample leakage from two sides of the channel or reaction zone when loading is excessive or microsphere concentration is too high, which would affect detection results. Both are produced by Shandong Micro-Diagnostic Biotechnology Co., Ltd.
   An RSV clinical sample S2 obtained from a relevant hospital.
   A fluorescence immunoassay analyzer produced by Shandong Micro-Diagnostic Biotechnology Co., Ltd., a timer (such as a stopwatch), and a pipette.
2. Coating sites
   Coating site one is located in the detection zone and is coated with an RSV antibody.
   Coating site two is located in the detection zone and is coated with a FluA antibody.
   Coating site three is located in the detection zone and is coated with a FluB antibody.
   Coating site four is located in the detection zone and is coated with a COVID-19 antibody.
   Coating site five is located in the quality control zone and is coated with a secondary antibody.
   A fluorescence labeling zone is fixed with dried fluorescence-labeled conjugated antibodies.
3. Detection method
   3.1 Four-test microfluidic chip coated with a hydrophobic material The microfluidic chip coated with a hydrophobic material (chip No. 6) was placed flat on a laboratory bench. 35 µL of RSV sample was added into a loading hole. After timing for 90 seconds, the control valve was used to make the water-absorbent material suck water for 3 minutes, and then the chip was interpreted using a fluorescence immunoassay analyzer, and detection results of the RSV sample were recorded. Each sample was repeatedly detected 3 times, whether there was a leakage phenomenon after loading was observed, and a detection signal value of each sample, a peak value of the quality control zone (C line), and a peak value of the detection zone (T line) were recorded.
   3.2 Four-test microfluidic chip before improvement The four-test microfluidic chip before improvement (chip No. 5) was placed flat on a laboratory bench. 35 µL of RSV sample was added into a loading hole of the microfluidic chip. After timing for 90 seconds, the control valve was used to make the water-absorbent material suck water for 3 minutes, and then the chip was interpreted using a fluorescence immunoassay analyzer, and detection results of the RSV sample were recorded. Each sample was repeatedly detected 3 times, whether there was a leakage phenomenon after loading was observed, and a detection signal value of each sample, a peak value of the quality control zone (C line), and a peak value of the detection zone (T line) were recorded.
4. Results
   As shown in Table 3, even if leakage occurs in the four-test microfluidic chip No. 6 coated with a hydrophobic material, it can be intercepted by the hydrophobic material, which will not lead to scattering of microspheres around the channel to affect a detection background, nor will it lead to leakage of liquid in the reaction zone to affect flow of microspheres toward the channel. When the water-absorbent material sucks water, the intercepted liquid can be sucked away without affecting detection results. Leakage also occurred in the four-test microfluidic chip No. 5 before improvement, and microspheres leaked in the reaction zone leading to invalid results. Although the water-absorbent material might also suck away the leaked liquid, nonspecific adsorption of microspheres at incorrect positions still affects interpretation results.

**Table 3 Sample detection results before and after improvement of the microfluidic chip**

| Chip No. 5 | | | | | |
|---|---|---|---|---|---|
| Sample | Item | Leakage Status | Detection Signal Value | C-line Peak Value | T-line Peak Value |
| S2 | RSV | Leakage | - | - | 74 |
| | | Leakage | - | - | 115 |
| | | No leakage | 311.58 | 62 | 100 |

| Chip No. 6 | | | | | |
|---|---|---|---|---|---|
| Sample | Item | Leakage Status | Detection Signal Value | C-line Peak Value | T-line Peak Value |
| S2 | RSV | Leakage, intercepted | 453.23 | 80 | 135 |
| | | No leakage | 332.32 | 109 | 185 |
| | | No leakage | 380.58 | 80 | 126 |

Experimental conclusion: There is a certain probability of leakage, and coating a hydrophobic material can effectively intercept leaked liquid to ensure an interpretation effect.

### Example 4

Experimental purpose: To verify the advantages of the dual-drive microfluidic chip's control zone contacting/detaching from the channel multiple times (more than twice) for detecting plasma sample IgE.
1. Material preparation
   Improved microfluidic chips No. 7 and No. 8 for IgE were used. A one-step method was used for chip No. 7 (the control zone contacted the channel once), and a two-step method was used for chip No. 8 (the control zone contacted the channel twice). Both were produced by Shandong Micro-Diagnostic Biotechnology Co., Ltd.
   IgE clinical plasma sample S3 was obtained from a relevant hospital.
   A fluorescence immunoassay analyzer produced by Shandong Micro-Diagnostic Biotechnology Co., Ltd., a timer (such as a stopwatch), and a pipette.
2. Coating sites
   Coating site one is located in the detection zone and is coated with an anti-human IgE antibody.
   Coating site two is located in the quality control zone and is coated with a biotinylated IgE antigen.
   The labeling zone is fixed with dried IgE fluorescence-labeled conjugated antibodies.
3. Detection method
   3.1 Dual-drive microfluidic chip where the filter in the control zone detaches from/contacts the channel twice to control the reaction process The IgE microfluidic chip (chip No. 8) was placed flat on a laboratory bench. 20 µL of IgE plasma sample was added into the loading hole. After waiting for 10 seconds, the plasma flowed through the detection zone and the quality control zone to reach the top of the channel. At this time, the control valve was used to make the water-absorbent material contact the channel to suck the excessive plasma until flow interruption occurred (first contact), allowing the plasma sample retained in the channel to react with the antibodies in the detection zone for 90 seconds. Then, 35 µL of goat serum was added into the loading hole as a buffer. After the buffer converged with the plasma in the channel, it continued to flow toward the control zone for about 10 seconds. After the plasma in the channel was sucked away and the fluorescent microspheres were dissolved to fill the channel, the control valve was used to disconnect the contact between the water-absorbent material and the channel (the water-absorbent material detached). After 2 minutes, the water-absorbent material was used to suck water again for 2 minutes (second contact). Then, the chip was interpreted using a fluorescence immunoassay analyzer, and detection results of the IgE sample were recorded. Each sample was repeatedly detected 3 times, and peak values of the quality control zone (C line) and the detection zone (T line) were recorded.
   3.2 Dual-drive microfluidic chip where the filter in the control zone detaches from/contacts the channel once to control the reaction process The IgE microfluidic chip (chip No. 7) was placed flat on a laboratory bench. 20 µL of plasma sample was added into a micro-loading hole. After waiting for 10 seconds, the sample flowed through the detection zone and the quality control zone. At this time, the control valve was used to make the water-absorbent material contact the channel to suck the excessive plasma until flow interruption occurred (first contact), allowing the plasma sample retained in the channel to react with the antibodies for 90 seconds. 35 µL of goat serum was added into the loading hole as a buffer. After the fluorescent microspheres were dissolved and flowed through the channel, they continuously entered the water-absorbent material in the control zone. After sucking water for 2 minutes, the chip was interpreted using a fluorescence immunoassay analyzer, and detection results of the IgE sample were recorded. Each sample was repeatedly detected 3 times, and peak values of the quality control zone (C line) and the detection zone (T line) were recorded.
4. Results
   As shown in Table 4, for the IgE of the two-step control microfluidic chip (chip No. 8) at 6 minutes, the C line peak values and T line peak values are relatively consistent. For the IgE of the one-step control microfluidic chip (chip No. 7), the detected C line peak value is low (which may be a result of the remaining IgE competing with the IgE antigen in area C to bind with the antibodies on the fluorescent microspheres), and the T line value of the two-step method is higher than that of the one-step method.

**Table 4 Sample detection results before and after improvement of the microfluidic chip**

| Chip No. 7 | | | |
|---|---|---|---|
| Sample | Item | C-line Peak Value | T-line Peak Value |
| S3 | IgE | 62 | 110 |
| | | 58 | 73 |
| | | 57 | 100 |

| Chip No. 8 | | | |
|---|---|---|---|
| Sample | Item | C-line Peak Value | T-line Peak Value |
| S3 | IgE | 85 | 258 |
| | | 96 | 274 |
| | | 88 | 266 |

Experimental conclusion: The detection results using two-step contact/detachment to control the reaction are significantly superior to the results of one-step control.

### Example 5

Experimental purpose: To contrastively verify the influence on interpretation results after coating a hydrophobic material and performing laser treatment on two sides of the positions of the upper cover channel corresponding to the substrate.
1. Material preparation
   A four-test (same test items as above) microfluidic chip No. 9 coated with a hydrophobic material and a microfluidic chip No. 10 after laser treatment. The difference is that chip No. 10 uses laser sintering at positions where chip No. 9 is coated with hydrophobic material. Both were produced by Shandong Micro-Diagnostic Biotechnology Co., Ltd.
   RSV clinical sample S2 was obtained from a relevant hospital.
2. Detection method
   2.1 Four-test microfluidic chip coated with a hydrophobic material The four-test microfluidic chip coated with a hydrophobic material (chip No. 9) was placed flat on a laboratory bench. 35 µL of RSV sample was added into a loading hole. A flow time of the sample in the micro-channel was recorded, followed by a time taken for the control valve to make the water-absorbent material suck water, and then the chip was left to stand for 90 minutes. Each sample was repeatedly detected 3 times, and whether there was a leakage phenomenon after loading was observed.
3.2 Four-test microfluidic chip with laser treatment The four-test microfluidic chip with laser treatment (chip No. 1 0) was placed flat on a laboratory bench. 35 µL of RSV sample was added into a loading hole. A flow time of the sample in the micro-channel was recorded, followed by a time taken for the control valve to make the water-absorbent material suck water, and then the chip was left to stand for 90 minutes. Each sample was repeatedly detected 3 times, and whether there was a leakage phenomenon after loading was observed.
4. Results
   As shown in Table 5, the liquid retention capacities of the four-test microfluidic chip No. 9 coated with a hydrophobic material and the four-test microfluidic chip No. 10 with laser treatment are consistent, and no leakage occurred in either. The laser-treated chip No. 10 does not affect the flow of liquid in the channel, and the required water absorption time is shorter.

**Table 5 Sample detection results before and after improvement of the microfluidic chip**

| **Chip No.** | **Flow Time** | **Water Absorption Time** | **Liquid retention** |
|---|---|---|---|
| **Chip No. 9** | **14s** | **2min24s** | **No leakage after standing for 90 min** |
| | **15s** | **2min43s** | **No leakage after standing for 90 min** |
| | **15s** | **2min55s** | **No leakage after standing for 90 min** |
| **Chip No. 10** | **16s** | **44s** | **No leakage after standing for 90 min** |
| | **16s** | **52s** | **No leakage after standing for 90 min** |
| | **15s** | **62s** | **No leakage after standing for 90min** |

Experimental conclusion: The liquid retention capacity of the hydrophobic surface after laser treatment is consistent with that of the chip coated with hydrophobic material. No leakage occurred after standing for 90 minutes, and it does not affect the flow of liquid in the channel, which was completed in about 15 seconds. At the same time, the hydrophobic surface formed by laser marking significantly shortens the subsequent water absorption time.

In known microfluidic chips, since a loading hole is arranged upstream of a labeling zone, a sample will first react through the labeling zone after passing through the loading hole and then enter a detection zone. For samples with high viscosity, the signal molecules in the labeling zone cannot be pushed to the detection zone by the sample itself, so a subsequent buffer is required for pushing. However, this method dilutes the sample, resulting in inaccurate detection results; meanwhile, other substances contained in the sample may also interfere with the signal molecules in the labeling zone.

In addition, the hook effect of immune reaction is also an urgent problem to be solved. The hook effect, also known as the HOOK effect, refers to a phenomenon of false negatives caused by an improper ratio of antigen to antibody, where the detection signal intensity decreases as the antigen concentration increases. This is because the target analyte occupies all reaction sites in the reaction zone, and the immune complex formed by the binding of the target analyte and the signal molecules reacts very little or not at all with the antigen/antibody in the detection zone, making it impossible for the detection zone to accurately measure a signal value.

In another aspect of the present invention, to address the defects in the prior art, the present invention provides a novel microfluidic chip and a detection method thereof. The present invention will be described in detail below in conjunction with the accompanying drawings. It should be noted that in the drawings, the same reference numerals are given to components having substantially the same or similar structures and functions, and repetitive descriptions thereof will be omitted.

As shown in FIG. 15 to FIG. 16, a microfluidic chip according to at least one embodiment of the present invention comprises a substrate 1, a cover plate 2, and a micro-channel 3 enclosed and formed by the substrate 1 and the cover plate 2. A groove provided in the cover plate 2 along its length direction L and the substrate 1 enclose to form the micro-channel. The micro-channel 3 comprises a buffer loading zone 31, a reaction zone 32, and a control zone 33. The buffer loading zone 31, the reaction zone 32, and the control zone 33 are sequentially in communication from left to right along the length direction L. A movable water-absorbent material 4 is arranged in the control zone 33. The water-absorbent material 4 can be moved to contact or be away from the reaction zone 32, and can be moved to contact the reaction zone 32 at a suitable time as needed to adsorb excessive sample in the micro-channel 3. The reaction zone 32 comprises a labeling zone 321, a detection zone 322, and a sample loading zone 323. The detection zone 322 and the sample loading zone 323 are both arranged on the same side of the labeling zone 321 (as shown in FIG. 15, on the right side of the labeling zone 321), so that after the sample enters the micro-channel through the sample loading zone 323, it first flows into the detection zone 322.

It should be noted that the length direction L in the drawings refers to the direction along the length of the microfluidic chip, which is exemplarily a horizontal rightward direction in the figures.

Specifically, a detection site and a quality control site are sequentially arranged on the detection zone 322 from left to right along the length direction L. The number of the detection site and the quality control site is one or more. The detection site is used to detect the presence or concentration of a target analyte, and the quality control site is used to verify the effectiveness of the reaction. Specifically, the labeling zone 321 comprises an antibody/antigen labeled with a signal molecule. The detection site of the detection zone 322 is coated with an antibody/antigen capable of binding with the target analyte. The target analyte is capable of simultaneously binding with the antibody/antigen of the labeling zone 321 and the detection site to form a double antigen/antibody sandwich complex. The quality control site of the detection zone 322 is coated with an antibody/antigen capable of binding with the antibody/antigen labeled with the signal molecule of the labeling zone.

A braking hole 331 is arranged on the right side of the control zone 33, which can at least partially extend to the control zone 33 to contact the water-absorbent material 4. The braking hole 331 is a rectangular through-hole penetrating through the substrate 1 and the cover plate 2. A through-hole at one end of the water-absorbent material 4 away from the reaction zone 32 is arranged within the braking hole 331 to allow for controlling the movement of the water-absorbent material 4. For example, a braking mechanism can be inserted into the braking hole 331 and pass through the through-hole on the water-absorbent material 4, thereby controlling the movement of the water-absorbent material 4 by driving the movement of the braking mechanism or controlling the movement of the microfluidic chip.

The water-absorbent material 4 may adopt polyester fiber, water-absorbent resin, water-absorbent gelatin, papermaking wood pulp, or other materials with water-absorbent characteristics.

The buffer loading zone 31 is provided with a buffer hole communicating the upper and lower surfaces of the cover plate 2, and the sample loading zone 323 is provided with a loading hole communicating the upper and lower surfaces of the cover plate 2. The buffer and the sample can be added into the micro-channel 3 through the buffer hole and the loading hole, respectively. The sample loading zone 323 and the labeling zone 321 are respectively arranged on two sides of the detection zone 322. Therefore, after the sample enters the micro-channel 3 through the sample loading zone 323 or the loading hole, it flows leftward to the detection zone 322 due to capillary action to bind with the detection zone 322, and the excessive sample can be adsorbed by the water-absorbent material 4.

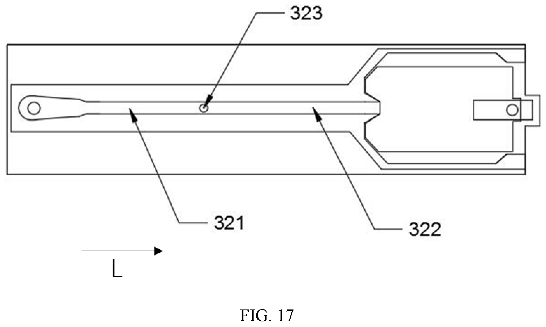

Alternatively, as shown in FIG. 17, the sample loading zone 323 may also be arranged between the labeling zone 321 and the detection zone 322. After the sample enters the micro-channel 3 through the sample loading zone 323 or the loading hole, it moves toward both the left and right ends of the micro-channel 3 due to capillary action. When flowing to the right, it flows to the detection zone 322. After the reaction is completed, the excessive sample is adsorbed by the water-absorbent material 4. However, this structure requires controlling the added amount of the sample so that the sample binds exactly with the detection zone 322 without binding with the labeling zone 321.

Exemplarily, the antigen/antibody labeled with a signal molecule can be an antigen/antibody molecule labeled with a signal molecule. The signal molecule can be, for example, a fluorescent protein, a fluorescent dye, fluorescent microspheres, or nanoparticles such as quantum dots.

On the other hand, the present invention further provides a detection method for a microfluidic chip, wherein the microfluidic chip as described above is applied for detection. The method comprises at least the following steps: 1) Adding a sample into the micro-channel 3 through the sample loading zone 323, and keeping the water-absorbent material 4 away from the reaction zone 32; 2) Waiting for a first time period, and after the sample flows back through the detection zone 322 under capillary action, moving the water-absorbent material 4 to contact the reaction zone 32 to suck excessive sample until flow interruption occurs, and allowing the sample retained in the micro-channel 3 to react with the detection zone 322; 3) After reacting for a second time period, adding a buffer into the micro-channel 3 through the buffer loading zone 31. The buffer passes through the labeling zone 321 under capillary action to wash the antigen/antibody coated with signal molecules in the labeling zone 321 and then enters the detection zone 322. When the signal molecules in the channel of the reaction zone 32 converge with the sample, moving the water-absorbent material 4 in the control zone 33 to contact the reaction zone 32 to adsorb the excessive sample into the control zone 33 through the water-absorbent material 4; 4) After the excessive sample in the channel is sucked away by the water-absorbent material 4 and the signal molecules of the labeling zone are dissolved to fill the channel of the reaction zone 32, moving the water-absorbent material 4 away from the reaction zone 32 to disconnect the contact between the water-absorbent material 4 and the channel of the reaction zone 32; 5) After reacting for a third time period, moving the water-absorbent material 4 in the control zone 33 to contact the detection zone 322 to adsorb the excessive liquid comprising the signal molecules into the control zone 33 through the water-absorbent material 4; 6) Finally, measuring a signal value of the detection zone 322. Exemplarily, a detector can be used to measure the signal value of the detection zone for subsequent analysis (the detector is a conventional technology, such as a fluorescence detector, as long as it is consistent with the signal carried by the antigen/antibody coated with signal molecules). Exemplarily, the first time period, the second time period, and the third time period can be adjusted according to requirements and sample conditions. For example, the first time period can be within a range of 0 to 60 seconds, the second time period can be within a range of 10 seconds to 300 seconds, and the third time period can be within a range of 30 seconds to 5 minutes.

The steps of the detection method can also be determined by a first determination condition to decide when to move the water-absorbent material 4 to contact the reaction zone 32 to suck the excessive sample until flow interruption occurs. The above example shows that a first time period has elapsed. Alternatively, the first determination condition may also include that a sample liquid volume is greater than or equal to a predetermined amount, wherein the predetermined amount is within a range of 15 µL to 50 µL; or a signal intensity of the signal molecules in the detection zone 322 is greater than or equal to a threshold higher than a reasonable background range of the chip. A plurality of experimental examples applying the microfluidic chip of the present invention are shown below to verify and illustrate the technical effects claimed by the present invention.

It should be noted that for the detection method proposed by the present invention, since the applied chip has both the detection zone and the sample loading zone arranged on the same side of the labeling zone, it is different from the loading method of conventional microfluidic chips (i.e., adding the sample and the buffer simultaneously, also known as the "one-step method"). The detection method proposed by the present invention requires adding the sample and the buffer separately, and requires the sample to flow into the detection zone after adding the sample, thus it can also be called a "two-step method". In the examples described below, the one-step method is used to represent conventional microfluidic chips (also known as chips before improvement), and the two-step method is used to represent the microfluidic chip proposed by the present invention (also known as the chip after improvement).

### Example 6

Experimental purpose: To respectively verify whether the one-step and two-step microfluidic chips can use whole blood as a detection sample.
1. Material preparation
   A microfluidic chip No. 1 before improvement and an IgE two-step microfluidic chip No. 2 after improvement. Compared with chip No. 1, chip No. 2 has a micro-loading hole added to the cover plate of the reaction zone channel 0.5 cm to the left of the control zone. Both were produced by Shandong Micro-Diagnostic Biotechnology Co., Ltd.
   IgE clinical whole blood sample S2 was obtained from a relevant hospital.
   A fluorescence immunoassay analyzer produced by Shandong Micro-Diagnostic Biotechnology Co., Ltd., a timer (such as a stopwatch), and a pipette.
2. Coating sites
   Coating site one is located at the detection site of the detection zone 322 and is coated with an IgE antibody.
   Coating site two is located at the quality control site of the detection zone 322 and is coated with a secondary antibody.
   The labeling zone 321 is fixed with dried IgE fluorescence-labeled conjugated antibodies.
3. Detection method
   3.1 Improved IgE two-step microfluidic chip The improved IgE two-step microfluidic chip was placed flat on a laboratory bench. 10 µL of whole blood sample was added into the micro-loading hole. After waiting for 10 seconds until the whole blood sample flowed back through the quality control site and the detection site, the water-absorbent material 4 was brought into contact with the channel to suck the excessive whole blood until flow interruption occurred, allowing the whole blood sample retained in the channel to react with the antibodies for 90 seconds. 35 µL of goat serum was added into the buffer hole as a buffer. After the buffer converged with the whole blood in the channel, water was sucked by the water-absorbent material 4 for 10 seconds. After the blood in the channel was sucked away and the signal molecules were dissolved to fill the channel, the contact between the water-absorbent material 4 and the channel was disconnected. After 2 minutes of reaction, water was sucked by the water-absorbent material 4 again for 2 minutes. Then, the chip was interpreted using a fluorescence immunoassay analyzer, and detection results of the IgE sample were recorded. Each sample was repeatedly detected 3 times, and signal values of the quality control site and the detection site were recorded.
   3.2 Microfluidic chip before improvement The microfluidic chip before improvement was placed flat on a laboratory bench. 35 µL of whole blood sample was added into the loading hole of the microfluidic chip to react for 2 minutes, then water was sucked by the water-absorbent material 4 for 3 minutes. Then, the chip was interpreted using a fluorescence immunoassay analyzer, and detection results of the IgE sample were recorded. Each sample was repeatedly detected 3 times, and signal values of the quality control site and the detection site were recorded.
4. Results
   As shown in Table 6, for the two-step microfluidic chip 2 at 6 minutes, the detection signal values, the quality control site peak values, and the detection site peak values are relatively consistent. For the one-step microfluidic chip 1, the detection results were invalid within 5 minutes because whole blood caused significant interference to the background, and the peak value of the quality control site could not be detected. Moreover, the detection site value of the two-step method is higher than that of the one-step method.

**Table 6 Sample detection results before and after improvement of the microfluidic chip**

| One-step Chip 1 | | | |
|---|---|---|---|
| Sample | Item | Quality Control Site Peak Value | Detection Site Peak Value |
| S2 | IgE | - | 153 |
| | | - | 189 |
| | | - | 230 |

| Two-step Chip 2 | | | |
|---|---|---|---|
| Sample | Item | Quality Control Site Peak Value | Detection Site Peak Value |
| S2 | IgE | 85 | 258 |
| | | 96 | 274 |
| | | 88 | 266 |

Experimental conclusion: The detection results using the two-step method are significantly superior to those of the one-step method. Adding whole blood in the one-step method leads to hemolysis, which contaminates the background with a red color and results in invalid detection results due to the failure to detect the quality control site. In the two-step method, the sample first reacts with the antibody, and then reacts with the signal molecules dissolved by the buffer, which can wash the whole blood sample in the channel clean without contaminating the background. It is feasible to use the two-step method for whole blood samples while the one-step method is not.

### Example 7

Experimental purpose: To contrastively verify whether the one-step and two-step methods for detecting IgE serum samples show the hook effect.
1. Material preparation
   A microfluidic chip No. 3 before improvement and an IgE two-step microfluidic chip No. 4 after improvement. Compared with chip No. 3, chip No. 4 has a micro-loading hole added to the cover plate of the reaction zone channel 0.5 cm to the left of the control zone. Both were produced by Shandong Micro-Diagnostic Biotechnology Co., Ltd.
   IgE clinical serum sample S3 (concentration > 5000 ng/ml) was obtained from a relevant hospital.
   A fluorescence immunoassay analyzer produced by Shandong Micro-Diagnostic Biotechnology Co., Ltd., a timer (such as a stopwatch), and a pipette.
2. Coating sites
   Coating site one is located at the detection site of the detection zone and is coated with an IgE antibody.
   Coating site two is located at the quality control site of the detection zone and is coated with a secondary antibody.
   The labeling zone is fixed with dried IgE fluorescence-labeled conjugated antibodies.
3. Detection method
   3.1 Improved IgE two-step microfluidic chip The improved IgE two-step microfluidic chip was placed flat on a laboratory bench. 10 µL of sample was added into the micro-loading hole. After waiting for 5 seconds until the sample flowed back through the quality control site and the detection site, the water-absorbent material 4 was brought into contact with the channel to suck the excessive sample until flow interruption occurred, allowing the sample retained in the channel to react with the antibodies for 90 seconds. 35 µL of goat serum was added into the buffer hole as a buffer. After the buffer converged with the whole blood in the channel, water was sucked by the water-absorbent material 4 for 10 seconds. After the residual blood in the channel was sucked away and the signal molecules were dissolved to fill the channel, the contact between the water-absorbent material 4 and the channel was disconnected. After 2 minutes of reaction, water was sucked by the water-absorbent material 4 again for 2 minutes. Then, the chip was interpreted using a fluorescence immunoassay analyzer, and detection results of the IgE sample were recorded. Each sample was repeatedly detected 3 times, and peak values of the quality control site and the detection site were recorded.
   3.2 Microfluidic chip before improvement The microfluidic chip before improvement was placed flat on a laboratory bench. 35 µL of sample was added into the loading hole for 2 minutes, and water was sucked by the water-absorbent material 4 for 3 minutes. Then, the chip was interpreted using a fluorescence immunoassay analyzer, and detection results of the IgE sample were recorded. Each sample was repeatedly detected 3 times, and peak values of the quality control site and the detection site were recorded.
   3.3 S3 sample was diluted 10-fold with goat serum, and the testing processes of 3.1 (chip No. 6) and 3.2 (chip No. 5) were repeated, and relevant data were recorded.
4. Results
   As shown in Table 7, by comparing the results of chip No. 5 and chip No. 3 using the one-step method, when the sample concentration increased 10-fold, the peak value of the detection site decreased instead, showing a hook effect. By comparing the results of chip No. 6 and chip No. 4 using the two-step method, when the sample concentration increased 10-fold, the peak value of the detection site increased accordingly, and no hook effect appeared.

**Table 7 Sample detection results before and after improvement of the microfluidic chip**

| One-step Chip 3 | | | |
|---|---|---|---|
| Sample | Item | Quality Control Site Peak Value | Detection Site Peak Value |
| S3 | IgE | 65 | 330 |
| | | 60 | 386 |
| | | 69 | 400 |

| Two-step Chip 4 | | | |
|---|---|---|---|
| Sample | Item | Quality Control Site Peak Value | Detection Site Peak Value |
| S3 | IgE | 137 | 466 |
| | | 146 | 514 |
| | | 159 | 471 |

| One-step Chip 5 | | | |
|---|---|---|---|
| Sample | Item | Quality Control Site Peak Value | Detection Site Peak Value |
| 10-fold Diluted S3 | IgE | 77 | 465 |
| | | 83 | 486 |
| | | 83 | 476 |

| Two-step Chip 6 | | | |
|---|---|---|---|
| Sample | Item | Quality Control Site Peak Value | Detection Site Peak Value |
| 10-fold Diluted S3 | IgE | 252 | 395 |
| | | 261 | 405 |
| | | 268 | 416 |

Experimental conclusion: No hook effect appears when detecting high-concentration samples with the two-step method, while the one-step method shows a hook effect. The two-step method provides more accurate detection results than the one-step method.

### Example 8

1. Material preparation
   A microfluidic chip No. 7 before improvement and an IgE two-step microfluidic chip No. 8 after improvement. Compared with chip No. 7, chip No. 8 has a micro-loading hole added to the cover plate of the reaction zone channel 0.5 cm to the left of the control zone. Both were produced by Shandong Micro-Diagnostic Biotechnology Co., Ltd.
   IgE clinical whole blood sample S1 was obtained from a relevant hospital.
   A fluorescence immunoassay analyzer produced by Shandong Micro-Diagnostic Biotechnology Co., Ltd., a timer (such as a stopwatch), and a pipette.
2. Coating sites
   Coating site one is located at the detection site of the detection zone 322 and is coated with an IgE antibody.
   Coating site two is located at the quality control site of the detection zone 322 and is coated with a secondary antibody.
   The labeling zone 321 is fixed with dried IgE fluorescence-labeled conjugated antibodies.
3. Detection method 3.1
   Improved IgE two-step microfluidic chip The improved IgE two-step microfluidic chip was placed flat on a laboratory bench. 6 µL of sample was added into the micro-loading hole. After timing for 30 seconds, 35 µL of goat serum was added into the buffer hole as a buffer. After the buffer converged with the whole blood in the channel, water was sucked by the water-absorbent material 4 for 10 seconds. After the blood in the channel was sucked away and the signal molecules were dissolved to fill the channel, the contact between the water-absorbent material 4 and the channel was disconnected. After 2 minutes of reaction, water was sucked by the water-absorbent material 4 again for 2.5 minutes. Then, the chip was interpreted using a fluorescence immunoassay analyzer, and detection results of the IgE sample were recorded. Each sample was repeatedly detected 3 times, and signal values of the detection site were recorded, and the average and deviation values were calculated.
   3.2 Microfluidic chip before improvement The microfluidic chip before improvement was placed flat on a laboratory bench. 10 µL of sample was diluted about 10-fold with a diluent (containing goat serum) and added into the loading hole for 2 minutes, and water was sucked by the water-absorbent material 4 for 3 minutes. Then, the chip was interpreted using a fluorescence immunoassay analyzer, and detection results of the IgE sample were recorded. Each sample was repeatedly detected 3 times, and signal values of the detection site were recorded, and the average and deviation values were calculated.
4. Results
   As shown in Table 8, for the IgE two-step microfluidic chip 8 at 6 minutes, the deviations of the detection results are all within 3. For the IgE microfluidic chip 7 at 5 minutes, the deviations of the detection results are within 7. Moreover, the signal value of the two-step method is higher than that of the one-step method. The deviation of the detection result after improvement is significantly smaller than the result before improvement. The difference in signal values due to the reaction sequence between the sample, the antibody, and the signal molecules indicates that the detection result of the improved chip is more accurate and the signal is stronger.

**Table 8 Sample detection results before and after improvement of the microfluidic chip**

| Chip 7 | | | | |
|---|---|---|---|---|
| Sample | Item | Detection Signal Value | Mean Value | Deviation |
| S1 | IgE | 186 | 179.67 | 6.81 |
| | | 183 | | |
| | | 170 | | |

| Chip 8 | | | | |
|---|---|---|---|---|
| Sample | Item | Detection Signal Value | Mean Value | Deviation |
| S1 | IgE | 280 | 280 | 3 |
| | | 277 | | |
| | | 283 | | |

### Example 9

1. Material preparation
   A four-test (RSV/FluA/FluB/COVID-19) microfluidic chip No. 9 before improvement and an improved four-test (same test items as above) two-step microfluidic chip No. 10. Compared with chip No. 9, chip No. 10 has a micro-loading hole added to the cover plate of the reaction zone channel 0.5 cm to the left of the control zone. Both were produced by Shandong Micro-Diagnostic Biotechnology Co., Ltd.
   RSV clinical sample S1 was obtained from a relevant hospital.
   A fluorescence immunoassay analyzer produced by Shandong Micro-Diagnostic Biotechnology Co., Ltd., a timer (such as a stopwatch), and a pipette.
2. Coating sites
   Coating site one is located at the detection site 1 of the detection zone 322 and is coated with an RSV antibody. Coating site two is located at the detection site 2 of the detection zone 322 and is coated with a FluA antibody. Coating site three is located at the detection site 3 of the detection zone 322 and is coated with a FluB antibody. Coating site four is located at the detection site 4 of the detection zone 322 and is coated with a COVID-19 antibody. Coating site five is located at the quality control site of the detection zone 322 and is coated with a secondary antibody.
   The fluorescence labeling zone 321 is fixed with dried fluorescence-labeled conjugated antibodies.
3. Detection method
   3.1 Improved four-test two-step microfluidic chip The improved four-test two-step microfluidic chip was placed flat on a laboratory bench. 35 µL of RSV sample was added into the micro-loading hole. After waiting for 0 seconds until the RSV sample flowed back through the quality control site and the detection site, the water-absorbent material 4 was brought into contact with the channel to suck the excessive sample until flow interruption occurred, allowing the RSV sample retained in the channel to react with the antibodies for 90 seconds. After the sample in the channel was sucked away and the signal molecules were dissolved to fill the channel, the contact between the water-absorbent material 4 and the channel was disconnected. After 2 minutes of reaction, water was sucked by the water-absorbent material 4 again for 2.5 minutes. Then, the chip was interpreted using a fluorescence immunoassay analyzer, and detection results of the RSV sample were recorded. Each sample was repeatedly detected 3 times, and signal values of the detection site 1 were recorded, and the average and deviation values were calculated.
   3.2 Four-test microfluidic chip before improvement The four-test microfluidic chip before improvement was placed flat on a laboratory bench. 35 µL of RSV sample was added into the loading hole for 2 minutes, allowing the sample retained in the channel to react with the antibodies, then water was sucked by the water-absorbent material 4 for 3 minutes. Then, the chip was interpreted using a fluorescence immunoassay analyzer, and detection results of the RSV sample were recorded. Each sample was repeatedly detected 3 times, and signal values of the detection site 1 were recorded, and the average and deviation values were calculated.
4. Results
   As shown in Table 9, for the improved four-test two-step microfluidic chip 10 at 6 minutes, the deviations of the RSV detection results are all within 3. For the four-test microfluidic chip 9 at 5 minutes, the deviations of the RSV detection results are within 4. Moreover, the signal value of the two-step method is higher than that of the one-step method. The deviation of the detection result after improvement is smaller than the result before improvement. The difference in signal values due to the reaction sequence between the sample, the antibody, and the signal molecules indicates that the detection result of the improved chip is more accurate and the signal is stronger.

**Table 9 Sample detection results before and after improvement of the microfluidic chip**

| Chip 9 | | | | |
|---|---|---|---|---|
| Sample | Item | Detection Signal Value | Mean Value | Deviation |
| S1 | RSV | 332.23 | 325.92 | 3.79 |
| | | 326.87 | | |
| | | 318.66 | | |

| Chip 10 | | | | |
|---|---|---|---|---|
| Sample | Item | Detection Signal Value | Mean Value | Deviation |
| S1 | RSV | 453.32 | 455.13 | 3 |
| | | 449.07 | | |
| | | 463 | | |

The above technical solutions only represent preferred technical solutions of the present invention. However, it should be understood by those skilled in the art that various modifications and improvements can be made to the above specific embodiments without departing from the concept of the present invention. **In** addition, various technical features and structures proposed in various aspects of the present invention can be combined in various ways without exceeding the protection scope of the present invention. The protection scope of the present invention is determined by the appended claims.

It should be noted that the embodiments in the present invention and features in the embodiments can be combined with each other in the absence of conflict.

**In** the description of the present invention, it should be understood that the orientation or positional relationships indicated by terms such as "center," "longitudinal," "transverse," "upper," "lower," "front," "rear," "left," "right," "vertical," "horizontal," "top," "bottom," "inner," and "outer" are based on the orientation or positional relationships shown in the drawings. These are only for the convenience of describing the present invention and simplifying the description, rather than indicating or implying that the referred device or element must have a specific orientation, or be constructed and operated in a specific orientation. Therefore, they cannot be understood as limiting the present invention. **In** addition, terms such as "first" and "second" are used only for descriptive purposes and cannot be understood as indicating or implying relative importance or implicitly specifying the number of indicated technical features. Thus, a feature defined by "first" or "second" may explicitly or implicitly include one or more such features. **In** the description of the present invention, "a plurality of" means two or more, unless otherwise specified.

**In** the description of the present invention, it should be noted that unless otherwise clearly specified and defined, terms such as "installation," "interconnection," "connection," and "arrangement" should be understood in a broad sense. For example, it may be a fixed connection, a detachable connection, or an integral connection ; it may be a mechanical connection or an electrical connection ; it may be a direct connection or an indirect connection through an intermediate medium, or it may be the internal communication between two elements. For those of ordinary skill in the art, the specific meanings of the above terms in the present invention can be understood according to specific circumstances.

## Claims

1. A microfluidic chip, comprising: a substrate (1); a cover plate (2) comprising a groove (21) extending along a length direction (L); and a micro-channel (3) enclosed and formed by the substrate (1) and the groove (21); wherein the groove (21) of the cover plate (2) comprises a channel area (211), the channel area (211) is sequentially provided with a loading area (2111), a reaction zone (2112), and a control zone (2113) along the length direction (L), and the loading area (2111), the reaction zone (2112), and the control zone (2113) are in fluid communication with each other; wherein a control valve (2113a) is arranged in the control zone (2113), the control valve (2113a) is configured to control a flow of liquid from the reaction zone (2112) toward the control zone (2113); and wherein a height of the reaction zone (2112) is lower than a height of the control zone (2113), and a width of the reaction zone (2112) is smaller than a width of the control zone (2113).

2. The microfluidic chip according to claim 1, wherein the control zone (2113) gradually narrows from its middle toward the reaction zone (2112) to facilitate contact with the liquid in the reaction zone and maintain a separated state.

3. The microfluidic chip according to claim 1, wherein the control valve (2113a) comprises: a movable fluid suction element (2113a1), the fluid suction element (2113a1) being capable of contacting or being away from the reaction zone (2112); and a braking hole (2113a3) arranged at a side away from the reaction zone (2112), wherein the braking hole (2113a3) is a rectangular through-hole penetrating through the substrate (1) and the cover plate (2), an end of the fluid suction element (2113a1) away from the reaction zone is provided with a through-hole (4), and the through-hole (4) is within the braking hole (2113a3).

4. The microfluidic chip according to claim 3, wherein the control zone (2113) is provided with a locking part (5) configured to maintain the fluid suction element (2113a1) at a position away from the reaction zone (2112).

5. The microfluidic chip according to claim 4, wherein the locking part (5) comprises a convex part arranged along a width direction (W), and the fluid suction element (2113a1) is correspondingly provided with a concave part matching the convex part.

6. The microfluidic chip according to claim 3, wherein a retention part (2113a2) is arranged at a junction between the control zone (2113) and the reaction zone (2112) on the cover plate (2), and the retention part (2113a2) at least partially extends into the reaction zone (2112) such that when the fluid suction element (2113a1) contacts a channel of the reaction zone (2112), at least a portion of the fluid suction element (2113a1) is inserted into the retention part (2113a2).

7. The microfluidic chip according to claim 6, wherein the retention part (2113a2) is a tooth part constructed as a downwardly recessed arc extending into the reaction zone (2112), a lower end of the arc is provided with a serrated or wavy structure, wherein a side of the fluid suction element (2113a1) close to the reaction zone (2112) gradually narrows, and a notch is provided in a middle of the side close to the reaction zone (2112) such that when the fluid suction element (2113a1) contacts the channel of the reaction zone (2112), the notch is inserted into the tooth part (2113a2).

8. The microfluidic chip according to claim 6, wherein the retention part (2113a2) is an inclined slot slanting toward the reaction zone (2112).

9. The microfluidic chip according to any one of claims 3 to 8, wherein the fluid suction element (2113a1) comprises a water-absorbent material, wherein the water-absorbent material comprises at least one of a desiccant, an antioxidant, or a moisture indicator.

10. The microfluidic chip according to claim 1, wherein the loading area (2111) is provided with a loading hole communicating upper and lower surfaces of the cover plate (2), and a height of the loading hole at the lower surface of the cover plate (2) is at the same level as the height of the reaction zone (2112).

11. The microfluidic chip according to claim 1, wherein the groove (21) is rectangular, a shape of the substrate (1) matches the groove (21), and the substrate (1) is placed within the groove (21) and is at the same level as a surface of the cover plate (2).

12. The microfluidic chip according to claim 1, wherein the channel area (211), a barrier area (212), and a pressing area (213) are respectively arranged from the middle toward the outer sides along a width direction (W) within the groove (21) of the cover plate (2), heights of the channel area (211) and the barrier area (212) are higher than a height of the pressing area (213) such that the substrate (1) is placed within the groove (21) on the lower surface of the cover plate (2) to contact the pressing area (213) and forms a gap with the channel area (211) and the barrier area (212).

13. The microfluidic chip according to claim 12, wherein the barrier area (212) is arranged at a periphery of the loading area (2111) and/or the reaction zone (2112) of the channel area (211), and in the length direction (L), the barrier area (212) covers at least a portion of the reaction zone (2112), or covers the loading area (2111) and/or the reaction zone (2112).

14. The microfluidic chip according to claim 12, wherein the substrate (1) is coated with a hydrophobic material at a position corresponding to the barrier area (212) in the groove (21) of the cover plate (2).

15. The microfluidic chip according to claim 14, wherein the reaction zone (2112) and the barrier area (212) are at the same level, and the barrier area (212) is also coated with the hydrophobic material.

16. The microfluidic chip according to claim 12, wherein the substrate (1) is subjected to laser treatment at a position corresponding to the barrier area (212) in the groove (21) of the cover plate (2) to form a hydrophobic surface.

17. The microfluidic chip according to claim 14 or 16, wherein the reaction zone (2112) and the barrier area (212) are at the same level, and the barrier area (212) is subjected to laser treatment to form a hydrophobic surface.

18. The microfluidic chip according to claim 14, wherein the height of the reaction zone (2112) is lower than the height of the barrier area (212) such that a sample does not enter the barrier area (212) due to gravity when flowing along the reaction zone (2112).

19. A microfluidic chip, comprising: a substrate (1); a cover plate (2); and a micro-channel (3) enclosed and formed by the substrate (1) and the cover plate (2); wherein the micro-channel (3) comprises a buffer loading zone (31), a reaction zone (32), and a control zone (33), the buffer loading zone (31), the reaction zone (32), and the control zone (33) are sequentially in communication along a length direction (L), a movable water-absorbent material (4) is arranged in the control zone (33), the water-absorbent material (4) is capable of contacting or being away from the reaction zone (32); and wherein the reaction zone (32) comprises a labeling zone (321), a detection zone (322), and a sample loading zone (323), and the detection zone (322) and the sample loading zone (323) are both arranged on the same side of the labeling zone (321).

20. The microfluidic chip according to claim 19, wherein the cover plate (2) is provided with a groove along the length direction (L), and the groove and the substrate (1) enclose to form the micro-channel (3).

21. The microfluidic chip according to claim 19, wherein a detection site and a quality control site are sequentially arranged in the detection zone (322) along the length direction (L), and the number of the detection site and the quality control site is one or more.

22. The microfluidic chip according to claim 19, wherein the labeling zone (321) comprises an antibody/antigen labeled with a signal molecule, the detection site of the detection zone (322) is coated with an antibody/antigen capable of binding with a target analyte, the target analyte is capable of simultaneously binding with the antibody/antigen of the labeling zone (321) and the detection site to form a double antigen/antibody sandwich complex, and the quality control site of the detection zone (322) is coated with an antibody/antigen capable of binding with the antibody/antigen labeled with the signal molecule of the labeling zone (321).

23. The microfluidic chip according to claim 19, wherein a braking hole (331) is arranged at a side of the control zone (33) opposite to the reaction zone (32), the braking hole (331) is a rectangular through-hole penetrating through the substrate (1) and the cover plate (2) and at least partially extends to the water-absorbent material (4) in the control zone (33), wherein an end of the water-absorbent material (4) away from the reaction zone is provided with a through-hole, and the through-hole is within the braking hole (331).

24. The microfluidic chip according to claim 19, wherein the buffer loading zone (31) is provided with a buffer hole communicating upper and lower surfaces of the cover plate (2), and the sample loading zone (323) is provided with a loading hole communicating the upper and lower surfaces of the cover plate (2).

25. The microfluidic chip according to claim 19, wherein the sample loading zone (323) and the labeling zone (321) are respectively arranged on two sides of the detection zone (322).

26. The microfluidic chip according to claim 19, wherein the sample loading zone (323) is arranged between the labeling zone (321) and the detection zone (322).

27. The microfluidic chip according to claim 22, wherein the antigen/antibody labeled with the signal molecule is an antigen/antibody molecule labeled with a signal molecule.

28. A detection method for a microfluidic chip, **characterized in that** the microfluidic chip according to any one of claims 1 to 9 is used for detection, and the method comprises at least the following steps: adding a sample into the micro-channel (3) through a sample loading zone (323), and keeping a water-absorbent material (4) away from a reaction zone (32); after the sample flows back through a detection zone (322) under capillary action and satisfies a first determination condition, moving the water-absorbent material (4) to contact the reaction zone (32) to suck excessive sample until flow interruption occurs, and allowing the sample retained in the micro-channel (3) to react with the detection zone (322); then, the water-absorbent material (4) separates from or continues to contact the reaction zone (32); after reacting for a second time period, adding a buffer into the micro-channel (3) through a buffer loading zone (31), the buffer passing through a labeling zone (321) under capillary action to wash the antigen/antibody coated with signal molecules in the labeling zone (321) and then entering the detection zone (322); when the signal molecules in a channel of the reaction zone (32) converge with the sample, moving the water-absorbent material (4) in a control zone (33) to contact the reaction zone (32) to suck excessive sample into the control zone (33) through the water-absorbent material (4); after the excessive sample in the channel is sucked away by the water-absorbent material (4) and the signal molecules of the labeling zone (321) are dissolved to fill the channel of the reaction zone (32), moving the water-absorbent material (4) away from the reaction zone (32) to disconnect the contact between the water-absorbent material (4) and the channel of the reaction zone (32); after reacting for a third time period, moving the water-absorbent material (4) in the control zone (33) to contact the detection zone (322) to suck excessive liquid comprising the signal molecules into the control zone (33) through the water-absorbent material (4); and measuring a signal value of the detection zone (322).

29. The detection method according to claim 28, wherein the first determination condition comprises: a first time period has elapsed, or a sample liquid volume is greater than or equal to a predetermined amount, or a signal intensity of signal molecules in the detection zone (322) is greater than or equal to a threshold.

30. The detection method according to claim 29, wherein the first time period is within a range of 0 to 60 seconds, and the predetermined amount is within a range of 15 µL to 50 ilL.

31. The detection method according to claim 28 or 29, wherein the second time period is within a range of 10 seconds to 300 seconds, and the third time period is within a range of 30 seconds to 5 minutes.
